# EUROPEAN PATENT APPLICATION

(11) **EP 2 977 461 A1**
(43) Date of publication of application: **27.01.2016**
(21) Application number: 15169975.8
(22) Date of filing: 05.11.2009
(51) Int. Cl.: C12P 21/04, C07K 14/745

(54) **METHOD OF PRODUCING SERUM-FREE INSULIN-FREE FACTOR VII**

(30) Priority: 12.11.2008 US 113792 P
(62) Divisional of application: 09826577.0
(71) Applicant: Baxalta Incorporated, Bannockburn, IL 60015 (US); Baxalta GmbH, 8152 Glattpark, Opfikon (CH)
(72) Inventor: von Fircks, Simone, 1210 Wien (AT); Elmer, Ruth, 2304 Orth an der Donau (AT); Dr. Reiter, Manfred, 1150 Wien (AT)
(74) Representative: Hoffmann Eitle

(57) **Abstract**

The invention provides methods and compositions for producing recombinant human factor VII (FVII) comprising obtaining a Chinese Hamster Ovary (CHO) cell line that expresses recombinant human FVII, culturing said CHO cell in serum-free media that lacks insulin wherein the production of said FVII in said CHO cell is comparable to the production of FVII in the presence of insulin.

## Description

### BACKGROUND OF THE INVENTION

The main role of FVII is to initiate the process of coagulation in conjunction with tissue factor (TF). Tissue factor is found on the outside of blood vessels and not normally exposed to the bloodstream. Upon vessel injury, tissue factor is exposed to the blood and circulating factor VII. Once bound to TF, FVII is activated to FVIIa by different proteases, among which are thrombin (factor IIa), activated factor X and the FVIIa-TF complex itself. The most important substrates for FVIIa-TF are Factor X and Factor IX. Factor VIIa which is present in the circulation in an amount corresponding to about 1% of the total Factor VII protein mass. Factor VII exists in plasma mainly as a single-chain zymogen which is cleaved by FXa into its two-chain, activated form, Factor VIIa.

Recombinant activated Factor VIIa (rFVIIa) has been developed as a prohaemostatic agent. The administration of rFVIIa offers a rapid and highly effective prohaemostatic response in haemophilic subjects with bleedings, who are limited in their treatment by other coagulation factors due to the development of antibodies. Also bleeding in subjects with Factor VII deficiency or subjects having a normal coagulation system but experiencing excessive bleeding can be treated successfully with rFVIIa.

The preparation of recombinant blood coagulation factors is gaining in importance. For example, Factor VIIa, like other clotting proteins, is subject to a variety of co- and post-translational modifications, including, e.g., asparagine-linked (N-linked) glycosylation; O-linked glycosylation; and γ-carboxylation of glutamic acid residues it is preferable to produce these recombinant proteins in mammalian cells which are able to modify the recombinant proteins appropriately.

This is to allow for the optimum growth of recombinant cells, mammalian cell culture, has traditionally been performed in the presence of animal serum or animal-derived components such as albumin, transferrin, insulin, etc. Nevertheless, recognizing that there is a need to reduce the risk of contamination and to decrease the variability in the product produced, various methods and compositions have been developed for use of serum-free media for the preparation of recombinant blood coagulation factors (see e.g., U.S. Patent 6,100,061 and US 2003/0203448A1 or US 2006/0094104A1). The use of such media in the preparation process also allows for a simpler purification of the expressed proteins. In most instances, recombinant cells are first cultured in serum-containing medium up to a high cell density, e.g. for a working cell bank, and subsequently they are re-adapted to serum-free medium during the production phase.

Methods for serum-free cultivation have produced variable results and have required the addition of insulin for cell growth and production of Factor VII proteins. Thus, there is a need in the art for methods for mammalian cell culture free of both serum or other animal-derived components and insulin to produce quantities of clotting proteins, particularly recombinant human Factor VII or Factor VII-related polypeptides.

### BRIEF SUMMARY OF THE INVENTION

According to specific aspects, the present invention relates to a method for producing recombinant human Factor VII (FVII) comprising obtaining a Chinese Hamster Ovary (CHO) cell line that expresses recombinant human FVII; and culturing the CHO cell in serum-free media that lacks insulin. Specifically, the production of the FVII in the CHO cell is comparable to the production of FVII in the presence of insulin.

The CHO cell used in the production method is prepared by adapting a CHO cell line that expresses recombinant human FVII modified for growth in serum-free culture to grow in cell culture in the absence of insulin by serially culturing the CHO cell line in decreasing amounts of insulin to obtain a FVII-producing CHO cell line that grows in the absence of insulin.

In specific embodiments, the CHO cell line that expresses recombinant human FVII modified for growth in serum-free culture is cell line 1E9 cell line deposited under Accession No. number 08100801 with the European Collection of Cell Cultures, Porton Down, U.K., on October 8, 2008.

In other embodiments, the FVII-producing CHO cell line produced to grow in the absence of insulin is a 1E9 cell line deposited under Accession No. number 08100802 with the European Collection of Cell Cultures, Porton Down, U.K., on October 8, 2008.

The cell culture medium used in the production method preferably comprises vitamin K1. More particularly, the media is a serum-free DMEM/HAM's F12 based formulation supplemented with one or more of the following additives i) glutamine; final concentration 0.9 g/l, ii) ferric sulfate x7H2O 0.0006 g/l, iv) putrescine, x 2HCl 0.0036g/l, vi) Vitamin K1 0.0025 g/l, vii) Synperonic; final concentration 1 g/l, Phenolred 0.008 g/l, Ethanolamine 0.00153g/l, and Na-hydrogencarbonate 2g/l).

The cells are prepared using a method in which the serially culturing comprises pulsing successive passages of CHO cells with decreasing concentrations of insulin. It was found that such pulsing produces viable cells whereas growing said cells in successively less insulin without the pulsing does not produce viable cells.

An exemplary step in the pulsing method comprises growing a CHO cell line up to day 14 in standard serum-free media that contains insulin and at day 14 transferring the cell line to a serum-free, insulin free media.

In a specific aspect, the cells are then grown in the serum-free, insulin free media for 1 to 3, preferably one day and then transferred again into an insulin-containing media that comprises 0.2 mg/ml insulin and grown in the insulin-containing media for 1 to 3, preferably 3 days. Subsequently, after growth for 1 to 3, but preferably, 3 days in insulin-containing media, the CHO cell line is again transferred into serum-free, insulin-free media.

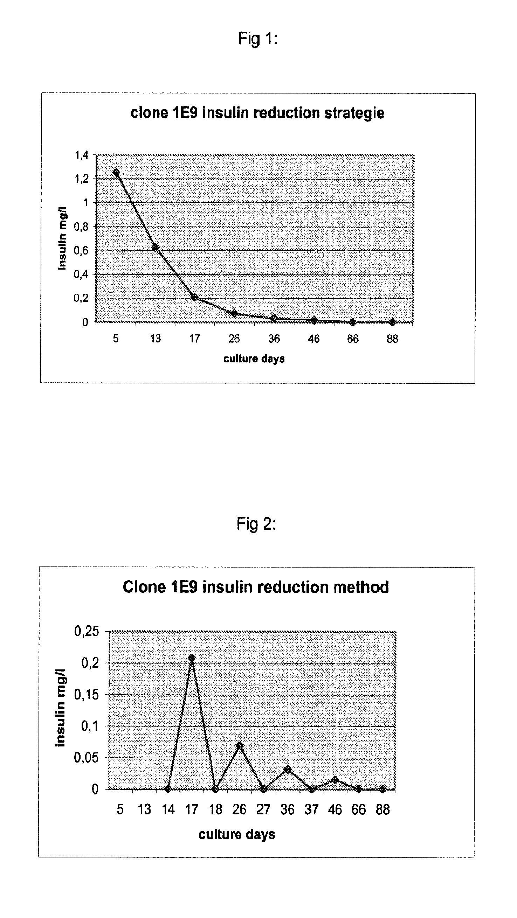

In exemplary embodiments, the cells are then grown in a serum-free, insulin free media for a further period of time, preferably one day and then transferred into an insulin-containing media that comprises 0.070 mg/ml insulin and grown in the insulin-containing media for 3 to 8, but preferably 8 days. After the growth for the 3 to 8, but preferably 8 days in insulin-containing media, the CHO cell line is transferred into serum-free, insulin-free media. The method then involves growing the cells in a serum-free, insulin free media for one day and then transferring the cells into an insulin-containing media that comprises 0.030 mg/ml insulin and growing the CHO cell line in the insulin-containing media for 3 to 9 days, but preferably, 9 days. Again, after growth for 3 to 9 days, but preferably 9 days in insulin-containing media, the CHO cell line is transferred into serum-free, insulin-free media. This cell line is then grown in a serum-free, insulin free media for one day and then transferring the cells into an insulin-containing media that comprises 0.016 mg/ml insulin and growing the CHO cell line in the insulin-containing media for3 to 9, but preferably 9 days. This cell line is then transferred to serum-free, insulin free media and is stable in insulin-free media for a least 30 passages and preferably at least 40 passages. The actual insulin concentration (based on calculation) during adaptation from day 0-88 is given in figure 1.

The invention thus also relates to a method of adapting a FVII-producing cell line that grows in serum-free media to a cell line that grows in serum-free, insulin free media, comprising pulsing successive passages of CHO cells with decreasing concentrations of insulin, wherein the pulsing comprises: a) growing the cell line for 3 to 9 days but more specifically 9 days in insulin-containing media followed by b)growth in insulin-free, serum-free media for one day and repeating steps a) and b) wherein the media in each successive step a) contains approximately half the concentration of insulin as the previous step a) until the media contains no detectable insulin.

In a preferred such adapting method, the method comprises a) growing a CHO cell line up to day 14 in standard serum-free media that contains insulin and at day 14 transferring the cell line to a serum-free, insulin free media; b) growing the cells of step b) in a serum-free, insulin free media for 1 to 3 days but more specifically, one day and then transferring the cells into an insulin-containing media that comprises 0.2 mg/ml insulin and growing the CHO cell line in the insulin-containing media for 1 to 3 days, but more specifically for 3 days; c) after growth for 1 to 3, but more specifically 3 days in insulin-containing media of step b), transferring the CHO cell line into serum-free, insulin-free media; d) growing the cells of step c) in a serum-free, insulin free media for one day and then transferring the cells into an insulin-containing media that comprises 0.070 mg/ml insulin and growing the CHO cell line in the insulin-containing media for 3 to 8 days but more specifically for 8 days; e) after growth for 3 to 8 days, but more specifically, for 8 days in insulin-containing media in step d), transferring the CHO cell line into serum-free, insulin-free media; f) growing the cells of step e) in a serum-free, insulin free media for one day and then transferring the cells into an insulin-containing media that comprises 0.030 mg/ml insulin and growing the CHO cell line in the insulin-containing media for 3 to 9 days, but more specifically for 9 days; g) after growth for one day in insulin-containing media in step f), transferring the CHO cell line into serum-free, insulin-free media; h) growing the cells of step g) in a serum-free, insulin free media for one day and then transferring the cells into an insulin-containing media that comprises 0.016 mg/ml insulin and growing the CHO cell line in the insulin-containing media for 3 to 9 days, but more specifically for 9 days; and i) after growth for the 3 to 9, but more specifically the 9 days in insulin-containing media in step h, transferring the CHO cell line into serum-free, insulin-free media, wherein the cells in step i) are adapted for long-term growth in serum-free, insulin-free media. The invention also relates to a cell produced according to these methods.

A further aspect of the invention relates to a recombinant CHO cell that expresses human FVII and is adapted for growth in an insulin-free media lacking animal-derived components. Preferably, the recombinant cell is produced by adapting a FVII-producing cell line that grows in serum-free media to a cell line that grows in serum-free, insulin free media, comprising pulsing successive passages of CHO cells with decreasing concentrations of insulin, wherein the pulsing comprises a) growing the cell line for 9 days in insulin-containing media followed by b) growth in insulin-free, serum-free media for one day and repeating steps a) and b) wherein the media in each successive step a) contains approximately half the concentration of insulin as the previous step a) until the media contains no detectable insulin.

A preferred recombinant CHO cell that expresses human FVII is one that is deposited under Accession No. number 08100801 with the European Collection of Cell Cultures, Porton Down, U.K., on October 8, 2008. A further preferred recombinant CHO cell that expresses human FVII is one that is deposited under Accession No. number 08100802 with the European Collection of Cell Cultures, Porton Down, U.K., on October 8, 2008.

Also contemplated is a method for large-scale production of a FVII or a FVII-related polypeptide in mammalian cells, the method comprising (a) inoculating an adapted recombinant cell of the invention into a culture vessel containing serum-free, insulin-free medium and propagating the mammalian cell culture at least until the cells reach a predetermined density; (b) transferring the propagated seed culture to a large-scale culture vessel containing serum-free insulin-free medium; (c) propagating the large-scale culture in serum-free insulin-free medium, at least until the cells reach a predetermined density; (d) maintaining the culture obtained in step (c) in serum-free insulin-free medium, under conditions appropriate for FVII expression or FVII-related polypeptide expression; and (e) recovering the FVII or the FVII-related polypeptide from the maintained culture.

Preferably, the method further comprises prior to step (b), repeating step (a) using seed culture vessels of progressively increasing size. The method also may further comprise maintaining the culture obtained in step (c) in serum-free, insulin-free media by regular harvesting of the culture medium and replacement by fresh medium.

The culture method may be any method such as for example, microcarrier process; a macroporous carrier process. The method may be a standard microcarrier process or a microcarrier perfusion process. Alternatively, the method is a suspension process, which may be a perfusion process or a batch/draw-fill process. The cell culture process may be a simple batch process, a fed-batch process or a draw-fill process. Prior to the inoculating step, the cells have been adapted to grow in serum-free medium lacking insulin. Further it is preferable that the cells, prior to the inoculating step, are capable of growing in suspension culture.

In the methods described, preferably, the desired FVII or FVII-related polypeptide is human FVII or a human FVII-related polypeptide. The large scale production method is one which preferably produces the FVII or a FVII -related polypeptide at a level at least about 1 mg/l/day of culture. More preferably, the FVII or a FVII-related polypeptide is produced at a level at least about 2.5 mg/l/day of culture. In other embodiments, the method yield is one in which FVII or a FVII-related polypeptide is produced at a level at least about 5 mg/l/day of culture. In other preferred embodiments, the FVII or a FVII related polypeptide is produced at a level at least about 8 mg/l/day of culture. In specific exemplary embodiments, the FVII or a FVII-related polypeptide is produced at a level of about 3-4 mg/l/day.

The method yields at least 5000U/L/day of FVII or FVII-related polypeptide. In other embodiments, the at least 7000 U/L/day of FVII or FVII-related polypeptide are produced. A preferred range of FVII or a FVII-related polypeptide units of activity produced is about 7000-10,000 U/l/day.

In specific embodiments, the method further comprises maintaining the culture obtained in step (c) in serum-free, insulin-free media by regular harvesting of part of the culture supernatant after sedimentation of the cell-containing carriers and replacement by fresh medium. Preferably, the method further comprises cooling of the culture to a pre-determined temperature below the setpoint of the cultivation before the sedimentation of carriers. For example, the culture is cooled to a temperature of from 5°C to 30°C below the temperature setpoint of the cultivation before the sedimentation of carriers. In other embodiments, the culture is cooled to a temperature of from 5°C to 20°C below the temperature setpoint of the cultivation. In still other embodiments, the culture is cooled to a temperature of from 5°C to 15°C below the temperature setpoint of the cultivation. More specific embodiments involve cooling the culture to a temperature of about 10°C below the temperature setpoint of the cultivation.

In defined aspects, the media used herein is a serum-free DMEM/HAM's F12 based formulation supplemented with one or more of the following additives i) glutamine; final concentration 0.9 g/l, ii) ferric sulfate x7H2O 0.0006 g/l, iv) putrescine, x 2HCl 0.0036g/l, vi) Vitamin K1 0.0025 g/l, vii) Synperonic; final concentration 1 g/l, Phenolred 0.008 g/l, Ethanolamine 0.00153g/l, and Na-hydrogencarbonate 2g/l).

In specific embodiments, the conditions in a preferred method are such that cell density of the cells in the large scale culture is at 1-3x10⁶/ml, and the culture is maintained at pH 7.1 +- 0.3, pO2 10-50% with a dilution rate of 0.25-1.5.

### BRIEF DESCRIPTION OF SEVERAL VIEWS OF THE DRAWINGS

Fig. 1 shows a graph of the reduction strategies for selecting a 1E9 cell line that grows in serum-free insulin-free conditions.
Fig. 2 shows a graph depicting the pulsing of CHO-K1 cells with serum-free medium with and without insulin to derive a CHO-K1 cell line able to grow in serum-free insulin-free media.
Fig. 3 shows a graph depicting the comparison of adaption of cells to serum-free insulin-free media by standard passaging dilution and pulsing technique of the present invention.

### DETAILED DESCRIPTION OF THE INVENTION

The present invention provides methods for producing recombinant human Factor VII (FVII) in a mammalian cell, for example a Chinese Hamster Ovarian (CHO) cells, under serum-free and insulin-free conditions. The method comprises obtaining a recombinant CHO cell line that expresses human FVII and culturing the CHO cell in serum-free media that lacks insulin.

The present invention encompasses cultivating mammalian cells expressing FVII or its derivatives in serum-free, insulin-free medium. As used herein, "serum-free" means that the media lacks any components that have been made in or extracted from an intact animal ("animal derived", for example, proteins isolated and purified from serum) or are components produced by using components that have been produced in an intact animal (for example, amino acids made by using an enzyme isolated and purified from an animal to hydrolyze a plant source material).

As used herein, "insulin free" or "medium lacking insulin" relates to media that contains no traceable amounts of insulin. Insulin includes both animal-derived insulin (e.g., purified from animal serum) and non-animal derived insulin, such as, for example, recombinant insulin produced in a yeast or bacterial cells or insulin produced in an established mammal cell line, such as, e.g., CHO, BHK or HEK cells.

Any cell culture medium that supports cell growth and maintenance under conditions of the invention may be used as long as that medium is free of (i.e., lacking) animal-derived components and free of insulin. Typically, medium contains water, an osmolarity regulator, a buffer, an energy source, amino acids, an inorganic or recombinant iron source, one or more synthetic or recombinant growth factors, vitamins, and cofactors. Media lacking animal-derived components and/or proteins are available from commercial suppliers, such as, for example, Sigma, JHR Biosciences, Gibco and Gemini.

In addition to conventional components, a medium suitable for producing Factor VII or a Factor VII-related polypeptide may advantageous contain Vitamin K. Vitamin K is a cofactor that is required for carboxylation of glutamic acid residues in Factor VII. Typically, Vitamin K may be present in the media at a concentration between about 0.1-50 mg/liter, preferably between about 0.5-25 mg/liter, more preferably between about 1-10 mg/liter and most preferably about 5 mg/liter.

The table below (Table 1) is shows a composition of an exemplary medium suitable for use in the present invention. The medium used is a serum-free medium (BCS). BCS medium is DMEM/HAM's F12 based medium that is exemplified as follows:

**Table 1**

| Component | g/L | Preferred amount |
|---|---|---|
| Synthetic minimum medium (DMEM/HAM's F12) | 1-100 | 11.00 -12.00 |
| L-glutamine | 0.05-1 | 0.9 |
| NaHCO3 | 0.1-10 | 2.00 |
| Ethanolamine | 0.0005-0.05 | 0.00153 |
| Synperonic F 68 | 0.01-10 | 1.0 |
| Phenolred | | 0.008 |
| Putrescine x 2HCl | | 0.0036 |
| ferric sulfate x7H₂O | | 0.0006 |
| Optionally: Insulin | | 0.005 |
| Vitamin K1 (or K3) | | 0.0025 |

Optionally, one or more of additional components may be added to the culture medium with suitable ranges listed. For example, the media may optionally contain Soybean peptone at a range of 0.5-50 g/L and preferably at 2.5 g/L. Another optional ingredient is Ascorbic acid present at range of 0.0005-0.05 g/L and preferably at a range of 0.0035g/L. In other media an optional ingredient is Na-selenite at a range of about 0.0001-0.001 g/L and preferably at 0.0086 g/L.

In practicing the present invention, the cells being cultivated are preferably mammalian cells, more preferably an established mammalian cell line, including, without limitation, CHO (e.g., ATCC CCL 61), COS-1 (e.g., ATCC CRL 1650), baby hamster kidney (BHK), and HEK293 (e.g., ATCC CRL 1573; Graham et al., J. Gen. Virol. 36:59-72, 1977) cell lines. A preferred CHO cell line is the CHO K1 cell line available from ATCC under accession number CCI61. In specific embodiments, the cell line used for the production methods described herein is CHO rFVIIa BI WCB07002 Accession number 08100801, this cell line is a recombinant Factor VII producing cell line that is adapted for growth in serum-free conditions.

Any suitable stable CHO K1 cell line expressing recombinant human FVII or derivatives may be adapted for growth under serum free and insulin free conditions and used in the practice of this invention. Suitably, a stable CHO K1 cell line expressing rFVII may be created by transfection with the plasmid vector pLN 194, plasmid vector encoding the cDNA of recombinant human FVII (rFVII) which has been previously described (Persson and Nielsen, 1996, FEBS Lett. 385, 241-243, incorporated by reference in its entirety); pLN329, a vector encoding a gamma-carboxylation recognition sequence, as described previously in PCT Application PCT/DK01/00634 (incorporated by reference in its entirety); or a combination thereof. Briefly, pLN174 vector carries the cDNA nucleotide sequence encoding human FVII including the propeptide under the control of a mouse metallothionein promoter for transcription of the inserted cDNA, and mouse dihydrofolate reductase cDNA under the control of an SV40 early promoter for use as a selectable marker. One such suitable cell line includes, but is not limited to, E11, disclosed in PCT Patent Application PCT/DK01/00634, incorporated herein in its entirety.

In the most suitable embodiment, the CHO K1 cell line expressing recombinant human FVII is adapted to grow in serum-free insulin-free medium, such as, for example, the cell line described in the examples below, including, but not limited to clone 1E9 derived from HHü07 DMCB#10 cell line. This cell line is referred to as CHO rFVIIa BI WCB07002 and was deposited in accordance with the Budapest Treaty as Accession number 08100801 with the European Collection of Cell Cultures, Porton Down, U.K., on October 8, 2008.

The present technology provides a method of adapting a recombinant CHO K1 cell line that expresses FVII to grow under serum-free and insulin-free conditions. In certain embodiments, such an adapted cell line is used for the production of rFVII. The CHO cell line is prepared by adapting a recombinant human FVII-expressing CHO cell line that is capable of growing in serum-free culture conditions to grow in a serum free cell culture in the absence of insulin by serially culturing said CHO cell line in decreasing amounts of insulin to obtain a FVII-producing CHO cell line that grows in the absence of insulin and in the absence of animal derived components. In some embodiments, the CHO cell line that grows in serum free culture media is 1E9. One embodiment of the method of serially culturing the human FVII-expressing CHO cell line comprises pulsing successive passages of the human FVII-expressing CHO cell line with decreasing concentrations of insulin. An exemplary CHO cell line that was prepared using the adaptation methods described herein is referred to as CHO rFVIIa BI insulinfree WCB08001 which was deposited in accordance with the Budapest Treaty as Accession number 08100802 with the European Collection of Cell Cultures, Porton Down, U.K., on October 8, 2008.

In an exemplary method, the human FVII-expressing CHO cell line (e.g., the cell line deposited as Accession number 08100801 with the European Collection of Cell Cultures, Porton Down, U.K., on October 8, 2008) is adapted for growth in insulin-free conditions by growing a CHO cell line that is capable of growth in serum-free media for up to day 14 in standard serum-free media that contains insulin and at day 14 transferring said cell line to a serum-free, insulin-free media. This step in the method is followed by growing the cells in a serum-free, insulin-free media for 1 to 3, but more specifically one day and then transferring said cells into an insulin-containing media that comprises 0.2 mg/ml insulin and growing the cells of the CHO cell line in said insulin-containing media for 1 to 3, but preferably for 3 days. The method may then further comprise after growth for the 1 to 3 days but preferably 3 days in insulin-containing media, transferring the cells of said CHO cell line into serum-free, insulin-free media. The method further comprises growing said cells of said CHO cell line in a serum-free, insulin free media for one day and then transferring said cells of said CHO cell line into an insulin-containing media that comprises 0.070 mg/ml insulin and growing said cells of said CHO cell line in said insulin-containing media for 3 to 8 days, but more specifically 8 days. The method can additionally include after growth for the 3 to 8 days but more specifically 8 days in insulin-containing media, transferring said cells of said CHO cell line into serum-free, insulin-free media. The method can further include growing said cells of said CHO cell line in a serum-free, insulin free media for one day and then transferring said cells of said CHO cell line into an insulin-containing media that comprises 0.030 mg/ml insulin and growing said cells of said CHO cell line in said insulin-containing media for 3 to 9 days, but more specifically 9 days. The method can also further include after growth for 3 to 9 days, but more specifically 9 day in insulin-containing media, transferring said cells of said CHO cell line into serum-free, insulin-free media. In another aspect, the method can further comprise growing said cells in a serum-free, insulin free media for one day and then transferring said cells into an insulin-containing media that comprises 0.016 mg/ml insulin and growing said CHO cell line in said insulin-containing media for 3 to 9 days, but more specifically 9 days. The cell line is then transferred into serum-free, insulin-free media, wherein the cells are adapted for long-term growth in serum-free, insulin-free media (culture day 66). In exemplary uses of this procedure, cell line CHO rFVIIa BI insulinfree WCB08001 (which was deposited in accordance with the Budapest Treaty as Accession number 08100802 with the European Collection of Cell Cultures, Porton Down, U.K., on October 8, 2008) was generated.

Further, the methods employed to adapt a FVII producing cell line to grow under serum-free insulin-free conditions may be applied to other cell lines. Other suitable cell lines include, without limitation, Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1); DUKX cells (CHO cell line) (Urlaub and Chasin, Proc. Natl. Acad. Sci. USA 77:4216-4220, 1980) (DUKX cells also being referred to as DXB11 cells), and DG44 (CHO cell line) *(*Cell, 33: 405, 1983, and Somatic Cell 10 and Molecular Genetics 12: 555, 1986). Also useful are 3T3 cells, Namalwa cells, myelomas and fusions of myelomas with other cells. In some embodiments, the cells may be mutant or recombinant cells, such as, e.g., cells that express a qualitatively or quantitatively different spectrum of enzymes that catalyze post-translational modification of proteins (e.g., glycosylation enzymes such as glycosyl transferases and/or glycosidases, or processing enzymes such as propeptides) than the cell type from which they were derived.

In other embodiments, cells from any mammalian source are engineered to express human Factor VII from a recombinant gene. As used herein, "Factor VII" or "Factor VII polypeptide" encompasses wild-type Factor VII (i.e., a polypeptide having the amino acid sequence disclosed in U.S. Patent No. 4,784,950), as well as variants of Factor VII exhibiting substantially the same or improved biological activity relative to wild-type Factor VII. The term "Factor VII" is intended to encompass Factor VII polypeptides in their uncleaved (zymogen) form, as well as those that have been proteolytically processed to yield their respective bioactive forms, which may be designated Factor VIIa.

Typically, Factor VII is cleaved between residues 152 and 153 to yield Factor VIIa. In the methods of the invention the Factor VII produced may be a wild-type Factor VII or it mayy be a Factor VII or Factor VIIa into which specific amino acid sequence alterations have been introduced that modify the bioactivity of the polypeptide to either increase or decrease the activity.

The biological activity of Factor VIIa in blood clotting derives from its ability to (i) bind to tissue factor (TF) and (ii) catalyze the proteolytic cleavage of Factor IX or Factor X to produce activated Factor IX or X (Factor IXa or Xa, respectively). For purposes of the invention, Factor VIIa biological activity may be quantified by measuring the ability of a preparation to promote blood clotting using Factor VII-deficient plasma and thromboplastin, as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. Alternatively, Factor VIIa biological activity may be quantified by (i) measuring the ability of Factor VIIa to produce of Factor Xa in a system comprising TF embedded in a lipid membrane and Factor X. (Persson et al., J. Biol. Chem. 272:19919-19924, 1997); (ii) measuring Factor X hydrolysis in an aqueous system; (iii) measuring its physical binding to TF using an instrument based on surface plasmon resonance (Persson, FEBS Letts. 413:359-363, 1997) and (iv) measuring hydrolysis of a synthetic substrate.

Factor VII variants having substantially the same or improved biological activity relative to wild-type Factor VIIa encompass those that exhibit at least about 25%, preferably at least about 50%, more preferably at least about 75% and most preferably at least about 90% of the specific activity of Factor VIIa that has been produced in the same cell type, when tested in one or more of a clotting assay, proteolysis assay, or TF binding assay as described above. Factor VII variants having substantially reduced biological activity relative to wild-type Factor VIIa are those that exhibit less than about 25%, preferably less than about 10%, more preferably less than about 5% and most preferably less than about 1% of the specific activity of wild-type Factor VIIa that has been produced in the same cell type when tested in one or more of a clotting assay, proteolysis assay, or TF binding assay as described above. Factor VII variants having a substantially modified biological activity relative to wild-type Factor VII include, without limitation, Factor VII variants that exhibit TF-independent Factor X proteolytic activity and those that bind TF but do not cleave Factor X.

In some embodiments, the cells used in practicing the invention are capable of growing in suspension cultures. As used herein, suspension-competent cells are those that can grow in suspension without making large, firm aggregates, i.e., cells that are monodispersed or grow in loose aggregates with only a few cells per aggregate. Suspension-competent cells include, without limitation, cells that grow in suspension without adaptation or manipulation (such as, e.g., hematopoietic cells or lymphoid cells) and cells that have been made suspension-competent by gradual adaptation of attachment dependent cells (such as, e.g., epithelial or fibroblast cells) to suspension growth.

In some embodiments, the cells used in practicing the invention are adhesion cells (also known as anchorage-dependent or attachment-dependent cells). As used herein, adhesion cells are those that need to adhere or anchor themselves to a suitable surface for propagation and growth.

### Culture Methods

The present invention provides methods for large-scale cultivation of mammalian cells, which are carried out by the steps of:
(i) inoculating cells into a seed vessel containing culture serum-free and insulin-free culture medium and propagating the seed culture at least until the cells reach a minimum predetermined density;
(ii) transferring the propagated seed culture to a large-scale culture vessel containing (a) serum-free insulin-free culture medium, under conditions in which the cells migrate onto the carriers (in case of a macroporous carrier process); and
(iii) propagating the large-scale culture in serum-free insulin-free medium, at least until said cells reach a useful density.

In some embodiments, the methods are carried out by the steps of:
(i) inoculating cells into a seed culture vessel containing serum-free and insulin-free culture media and propagating the seed culture at least until the cells reach a minimum predetermined density;
(ii) transferring the propagated seed culture to a large-scale culture vessel containing (a) serum-free and insulin-free culture medium and (b) macroporous carriers, under conditions in which the cells migrate into the carriers; and
(iii) propagating the large-scale culture in serum-free insulin-free medium, at least until said cells reach a useful density.

In some embodiments, the methods further comprise the step of:
(iv) maintaining the culture obtained in step (iii) in serum-free and insulin-free medium by regular harvesting of the culture medium and replacement by fresh serum-free insulin-free medium.

Medium exchange is performed by allowing the microcarriers to settle to the bottom of the culture vessel, after which a predetermined percentage of the medium is removed and a corresponding percentage of serum-free insulin-free media is added to the culture. The microcarriers are then resuspended in the medium. This process of medium removal and replacement can be repeated at predetermined intervals, such as, for example, every 24 hours.

In some embodiments, an additional optional step includes cooling step within 10-240 minutes, such as, e.g., 20-180 minutes, or 30-120 minutes, before sedimenting the cell-containing microcarriers. The step is typically carried out as follows: The bioreactor is cooled and the temperature is monitored. When the bioreactor reaches a pre-determined temperature below the setpoint temperature, such as, e.g., 10°C below the set point of the culturing, the stirring of the bioreactor contents is stopped and the cell-containing carriers are sedimented. When media exchange has taken place, the temperature is again regulated to the setpoint of the culturing. The fresh media being added is typically pre-warmed to a temperature close to the setpoint of the cultivation.

In some embodiments of the invention, the process is a microcarrier process and the cells are anchorage dependent cells. In such embodiments, both the propagation phase and the production phase include the use of microcarriers. The anchorage dependent cells should be able to migrate unto the carriers (and into the carriers if a macroporous carrier is used) during the propagation phase(s) and to migrate to new carrier when being transferred to the production bioreactor. If the anchorage dependent cells are not sufficiently able to migrate to new carriers by themselves, they can be liberated from the carriers by contacting the cell containing microcarriers with proteolytic enzymes or EDTA. The medium used (specifically, the serum-free and insulin-free medium) should furthermore contain components suitable for supporting the growth of anchorage-dependent cells; such media for anchorage dependent cells is readily available from commercial suppliers, such as, e.g., Sigma.

If suspension-adapted or suspension-competent cells are used in a microcarrier process, the propagation of cells may be done in suspension, thus microcarriers are only needed in the production phase.

### Inoculation and initial propagation:

It will be understood that step (i) may be repeated with a progressive increase in the size of the seed culture vessel, until a sufficient number of cells is obtained for step (ii). For example, one or more seed culture vessels of 5 liter, 50 liter, 100 liter, or 500 liter may be used sequentially. A seed culture vessel as used herein is one that has a capacity of between about 5 liter and 1000 liter. Typically, cells are inoculated into a seed culture vessel at an initial density of about 0.2-0.4 x 10⁶ cells/ml and propagated until the culture reaches a cell density of about 1.0 x 10⁶ cells/ml. As used herein, a minimum density is between about 0.8 and about 1.5 x 10⁶ cells/ml.

### Microcarriers:

As used herein, microcarriers are particles, often cellulose- or dextran-based, which typically are small enough to allow them to be used in suspension cultures (with a stirring rate that does not cause significant shear damage to cells); are solid, porous, or have a solid core with a porous coating on the surface; and have positively charged surfaces (exterior and interior surface in case of porous carriers). Typically, the microcarriers have an overall particle diameter between about 150 and 350 um; and have a positive charge density of between about 0.8 and 2.0 meq/g. Exemplary such microcarriers include, without limitation, Cytodex 1TM and Cytodex 2TM (Amersham Pharmacia Biotech, Piscataway NJ).

In one series of embodiments, the microcarrier is a solid carrier. Solid carriers are particularly suitable for anchorage-dependent cell growth. In another series of embodiments, the microcarrier is a macroporous carrier.

Macroporous carriers: As used herein, macroporous carriers are particles, usually cellulose-based, which have the following properties: (a) they are small enough to allow them to be used in suspension cultures (with a stirring rate that does not cause significant shear damage to cells); (b) they have pores and interior spaces of sufficient size to allow cells to migrate into the interior spaces of the particle and (c) their surfaces (exterior and interior) are positively charged. In one series of embodiments, the carriers: have an overall particle diameter between about 150 and 350 µm; have pores having an average pore opening diameter of between about 15 and about 40 µm; and have a positive charge density of between about 0.8 and 2.0 meq/g. In some embodiments, the positive charge is provided by DEAE (N, N,-diethylaminoethyl) groups. Useful macroporous carriers include, without limitation, Cytopore 1TM and Cytopore 2TM (Amersham Pharmacia Biotech, Piscataway NJ). Particularly preferred are Cytopore 1TM carriers, which have a mean particle diameter of 230 um, an average pore size of 30 µm, and a positive charge density of 1.1 meq/g.

### Large-scale culture conditions:

As used herein, a large-scale culture vessel has a capacity of at least about 100 liters, preferably at least about 500 liters, more preferably at least about 1000 liters and most preferably at least about 5000 liters. Typically, step (ii) involves transferring about 50 liters of the propagated seed culture (having about 1.0 x 10⁶ cells/ml) into a 500 liters culture vessel containing 150 liters of culture medium. The large-scale culture is maintained under appropriate conditions of, e.g., temperature, pH, dissolved oxygen tension (DOT), O₂ and CO₂ tension, and agitation rate, and the volume is gradually increased by adding medium to the culture vessel.

In case of a microcarrier process the culture vessel also comprises about 750 g microcarriers. After the transfer, the cells typically migrate onto the surface of the carriers within the first 24 hours. In case of a macroporous carrier process the culture vessel also comprises about 750 g macroporous carriers. After the transfer, the cells typically migrate into the interior of the carriers within the first 24 hours.

The term "large-scale process" may be used interchangeably with the term "industrial-scale process". Furthermore, the term "culture vessel" may be used interchangeably with "tank", "reactor", and "bioreactor".

### High-level protein expression:

When the cells are being propagated in order to produce high levels of a Factor VII protein, the period of time until the cell density reaches a predetermined cell density (e.g., at least about 1x10⁶ cells/ml) is designated the "growth phase". The growth phase normally comprises the steps (i), (ii) and (iii). When the cell density reaches the predetermined value (e.g., at least about 1x10⁶ cells/ml, preferably at least 2x10⁶ cell/ml, more preferred 5x10⁶ cells/ml), the phase is designated the "production phase". The production phase normally comprises step (iv). Any suitable, connected parameter changes are introduced at this stage.

### Culture vessels:

The culture vessels may be e.g. conventional stirred tank reactors (CSTR) where agitation is obtained by means of conventional impeller types or airlift reactors where agitation is obtained by means of introducing air from the bottom of the vessel. Among the parameters controlled within specified limits are pH, dissolved oxygen tension (DOT), and temperature. The pH may be controlled by e.g. varying the CO₂ concentration in the headspace gas and by addition of base to the culture liquid when required. Dissolved oxygen tension may be maintained by e.g. sparging with air or pure oxygen or mixtures thereof. The temperature-control medium is water, heated or cooled as necessary. The water may be passed through a jacket surrounding the vessel or through a piping coil immersed in the culture.

Once the medium has been removed from the culture vessel, it may be subjected to one or more processing steps to obtain the desired protein, including, without limitation, centrifugation or filtration to remove cells that were not immobilized in the carriers; affinity chromatography, hydrophobic interaction chromatography; ion-exchange chromatography; size exclusion chromatography; electrophoretic procedures (e.g., preparative isoelectric focusing (IEF), differential solubility (e.g., ammonium sulfate precipitation), or extraction and the like. (see, generally, Scopes, Protein Purification, Springer-Verlag, New York, 1982; and Protein Purification, J.C. Janson and Lars Ryden, editors, VCH Publishers, New York, 1989).

Purification of Factor VII or Factor VII-related polypeptides may involve, e.g., affinity chromatography on an anti-Factor VII antibody column (see, e.g., Wakabayashi et al., J. Biol. Chem. 261:11097, 1986; and Thim et al., Biochem. 27:7785, 1988) and activation by proteolytic cleavage, using Factor Xlla or other proteases having trypsin-like specificity, such as, e.g., Factor IXa, kallikrein, Factor Xa, and thrombin. (see, e.g., Osterud et al., Biochem. 11:2853 (1972); Thomas, U.S. Patent No. 4,456,591; and Hedner et al., J. Clin. Invest. 71:1836 (1983)). Alternatively, Factor VII may be activated by passing it through an ion-exchange chromatography column, such as Mono Q® (Pharmacia) or the like.

The present invention further provides methods for large-scale cultivation of mammalian cells to produce industrial amounts of Factor VII polypeptides that are expressed by such cells under serum-free and insulin-free conditions. The methods are carried out by the steps of:
(i) inoculating a cells into a culture vessel containing serum-free insulin-free medium and propagating said mammalian cell culture at least until the cells reach a minimum predetermined density;
(ii) transferring said propagated cell culture to a large-scale culture vessel containing serum-free insulin-free culture medium; and
(iii) propagating the large-scale culture in serum-free insulin-free medium at least until said cells reach a useful density.

In some embodiments, the methods further comprise the step of:
(iv) maintaining the culture obtained in step (iii) in serum-free insulin-free medium by regular harvesting of the culture medium and replacement by fresh serum-free insulin-free medium.

The following description of processes is applicable for any cell type in any formulation of serum-free insulin-free medium. The first two processes described are for cells attached to and/or immobilized in a macroporous carrier.

### Microcarrier Processes:

Two types of microcarrier processes may be used, which are standard microcarrier process and microcarrier perfusion process. In the standard microcarrier process, the process is operated in two distinct phases, the growth phase and the production phase.

### Growth Phase

In a standard microcarrier-process the cells are inoculated into a seed culture vessel containing serum-free, insulin-free culture medium and propagated until the cells reach a minimum density. Subsequently, the propagated seed culture is transferred to a large-scale culture vessel containing (a) serum-free, insulin-free culture medium and (b) microcarriers, under conditions in which the carriers are fully colonized by the cells, for example by migrating into the carriers in case of a process using macroporous carriers.

In this growth phase, the cells are grown on microcarriers until the carriers are fully colonized. Medium exchange is performed by allowing the microcarriers to settle to the bottom of the culture vessel, after which a predetermined percentage of the tank volume is removed and a corresponding percentage tank volume of fresh serum-free insulin-free medium is added to the vessel. The microcarriers are then resuspended in the medium and this process of medium removal and replacement are repeated at a predetermined interval, for example every 24 hours. The amount of replaced medium depends on the cell density and may typically be from 10-95%, preferably from 25% to 80%, of the tank volume as shown in Table 2 below.

When the cell density reaches the value suitable for protein expression, about 60-95% of the medium in the tank is changed every 24 hours, preferably about 80%. An 80% medium exchange is also preferably used in the production phase.

An outline of this aspect of the process is shown in the following Table 2:

**Table 2**

| **Setpoint** | **Range** | **Preferred range** | **More preferred Value** |
|---|---|---|---|
| pH | 6-8 | 6.6-7.6 | 7.0 |
| Temperature | 28-40°C | 34-38°C | 36-37°C |
| Dissolved Oxygen Tension | 10- 90% of saturation | 20-80% of saturation | 50% of saturation |
| Daily Medium Change: | | | |
| - % of medium changed at | 10-35% of medium exchanged at 0.4-1.0x10⁶ cells/ml | 25 % of medium exchanged at 0.4-1.0x10⁶ cells/ml | 25% of medium exchanged at 0.5x106 cells/ml |
| - % of medium changed at | 30-70% of medium exchanged at 0.7-3.0x10⁶ cells/ml | 50% of medium exchanged at 0.7-3.0x10⁶ cells/ml | 50% of medium exchanged at 1.0x106 cells/ml |
| - % of medium changed at | 60-90% of medium exchanged at 1.0-12.0x10⁶ cells/ ml | 80% of medium exchanged at 1.0-12.0x10⁶ cells/ml | 80% of medium exchanged at 2.0-10x10⁶ cells/ml |

Some of the setpoints that are suitable for production of FVII are not necessarily suitable for the initial growth of the cells, either in seed culture or on the microcarriers. For example, temperature, DOT, and/or pH may be different for the two phases.

The medium exchange at this stage, even if at the same level as in the production phase, is done to keep the cells alive and growing.

### Production Phase

In the growth phase the culture is propagated until the cells reach a density of 1-12x10⁶ cells per ml. Reaching this density, the culture enters the production phase. Setpoints may also be changed at this point and set at values suitable for production of FVII. The medium exchange is performed by allowing the microcarriers to settle to the bottom of the tank, after which the selected % of the tank volume is removed and a corresponding % tank volume of fresh medium is added to the vessel. From 25-90% of the tank volume may be exchanged; preferably, 80% of the tank volume is exchanged. The microcarriers are then resuspended in the medium and this process of medium removal and replacement are repeated every 10-48 hours; preferably, every 24 hours.

An outline of this aspect of the process is shown in Table 3.

**Table 3**

| **Setpoint** | **Range** | **Preferred range** | **More preferred Value** |
|---|---|---|---|
| pH | 6-8 | 6.6-7.6 | 7.0 for CHO |
| Temperature | 26-40°C | 30-37°C | 32°C for CHO |
| Dissolved Oxygen Tension | 10-90% of saturation | 20-80% of saturation | 50% |
| -% of medium changed | 25-90% of medium exchanged every 10-48 hours | 80% of medium changed every 10-48 hours | 80% of medium changed every 24 hours |

Optionally, a drop in temperature set point of the cultivation may be employed when entering and during the production phase. In particular, a drop in temperature is preferred when using a CHO cell line. Temperature ranges and preferred values in growth and production phase, respectively, can be seen from Tables 2 and 3. A temperature of about 32°C would be preferred for a CHO cell line during the production phase.

Alternatively, a cooling step may optionally be applied immediately before each sedimentation of carriers. The culture is cooled to a predetermined temperature below the temperature setpoint of the cultivation (e.g. from 5°C to 30°C, or from 5°C to 20°C, or from 5°C to 15°C, or to about 10°C below setpoint). The cooling step is done within 10-240 minutes, such as, e.g, 20-180 minutes, or 30-120 minutes, before sedimenting the cell-containing microcarriers. The step is typically carried out as follows: the bioreactor is cooled and the temperature is monitored. When the bioreactor reaches a pre-determined temperature below the setpoint temperature, such as, e.g., 10°C below the set point of the culturing, the stirring of the bioreactor contents is stopped and the cell-containing carriers are sedimented. When media exchange has taken place, the temperature is again regulated to the setpoint of the culturing. The fresh media being added is typically prewarmed to a temperature close to the setpoint of the cultivation.

### Microcarrier Perfusion Process:

This process resembles the standard microcarrier process and is again operated in two distinct phases, the growth phase and the production phase. The main difference between this and the standard process described above is the method employed to change the culture medium. In the previously described standard microcarrier process a defined percentage of the tank volume, for example, 80% of the total tank volume, is changed all at once. In a perfusion process the medium is added continuously and an equal volume of harvest is also removed continuously. Essentially, the medium (defined % tank volume) is changed gradually over a predetermined period of time, for example a 24-hour period.

The microcarriers are kept in the vessel by using a separation device (or perfusion device) that allows the medium to leave the vessel but retains the microcarriers within the tank.

### Growth Phase

As described for standard microcarrier process except for the gradual medium exchange. The exchange of medium is given as % tank volume per day, i.e., 24 hours. An outline of this aspect of the process is shown in Table 4.

**Table 4**

| **Setpoint** | **Range** | **Preferred range** | **More preferred Value** |
|---|---|---|---|
| pH | 6-8 | 6.6-7.6 | 7.0 |
| Temperature | 28-40°C | 34-38°C | 36-37°C |
| Dissolved Oxygen Tension | 10-90% of saturation | 20-80% of saturation | 50% |
| Medium Flow Rate | | | |
| - % tank volume per day (24 hours) at | 10-35% of medium perfused at 0.4-1.0x10⁶ cells/ml | 25% of medium perfused 0.4-1.0x10⁶ cells/ ml | 25% of medium perfused at 0.5x10⁶ cells ml-1 |
| - % tank volume per day | 30-70% of medium per | 50% of medium per- | 50% of medium perfused |
| (24 hours) at | fused at 0.7-3.0x10⁶ cell ml | fused at 0.7-3.0x10⁶ cells/ml | at 1.0x10⁶ cells/ml |
| - % tank volume per day (24 hours) at | 60-95% of medium perfused at 1.0-12.0x10⁶ cells/ml | 80% of medium perfused at 1.0-12.0x10⁶ cells/ml | 80% of medium perfused at 2.0-10x106 cells/ml |

Again, even though the culture is being perfused at high medium exchange (e.g., 80% tank volume) at an early stage this is not considered to be the production phase. This is because some of the setpoints that are suitable for production of FVII may not be suitable for the initial growth of the cells on the microcarriers. The perfusion with fresh medium at this stage is done to keep the cells alive and growing. For the purposes of comparison with the 'standard microcarrier process' the flow rate is expressed in terms of percentage tank volume of medium per day (24 hours).

### Production Phase

As in the above-described process, in the growth phase the culture is propagated until the cells reach a density of 1-12x10⁶ cells per ml. Reaching this density, the culture enters the production phase.

The medium perfusion is performed continuously. For the purposes of comparison with the 'standard microcarrier process' the flow rate of medium is expressed in terms of percentage tank volume of medium per defined period of time. Medium perfusion may be from 25-90% tank volume per 10-48 hours; preferably, the medium perfusion is 80% per 10-48 hours, more preferred 80% tank volume every 24 hours. An outline of this aspect of the process is shown in Table 5.

**Table 5**

| **Setpoint** | **Range** | **Preferred range** | **More preferred Value** |
|---|---|---|---|
| pH | 6-8 | 6.6-7.6 | 7.0 for CHO |
| Temperature | 26-40°C | 30-37°C | 32°C for CHO |
| Dissolved Oxygen Tension | 10-90% | 20-80% | 50% |
| -% tank volume of medium perfused | 25-90% of medium perfused every 10-48 hours | 80% of medium perfused every 10-48 hours | 80% of medium perfused every 24 hours |

Suitable means for achieving retention of carriers is a settling device inside the vessel, e.g. a dip tube.

A drop in temperature may be employed when entering - and during - the production phase. In particular when using a CHO cell line, a drop in temperature is preferred. Temperature ranges and preferred values in growth and production phase, respectively, can be seen from Tables 2 and 3. A temperature of about 32°C would be preferred for a CHO cell line during the production phase.

### Suspension Cell Processes:

There are two main options for a suspension cell process which are: 1) Perfusion Process; and 2) Batch / Draw-Fill Process.

### Perfusion Process:

This process resembles the process outlined for microcarrier perfusion. The main difference is 1) that the cells are grown freely suspended without being immobilized in carriers and 2) the nature of the perfusion device employed to retain the cell in the culture vessel. The process is again operated in two distinct phases: the growth phase and production phase.

### Growth Phase

In a suspension cell-perfusion process the cells are inoculated into a seed culture vessel containing serum-free insulin-free culture medium and propagated until the cells reach a minimum predetermined density. Subsequently, the propagated seed culture is transferred to a large-scale culture vessel containing serum-free insulin-free culture medium and propagated until at least a predetermined cell density is reached. In this phase the cells are grown in suspension to allow the cell number within the culture vessel to increase to a predetermined or critical value. The medium exchange is performed by continuously perfusing the culture vessel with fresh medium. The amount of perfused medium depends on the cell density and may typically be from 10-95%, preferably from 25% to 80%, of the tank volume per day (24 hours) as shown in Table 6 below. When the cell density reaches the value suitable for initiation of production phase, 60-95% of the tank medium in the tank is changed every 24 hours, preferably 80%. An 80% medium exchange is also preferably used in the production phase.

Again, even though the culture is being perfused at an early stage this is not considered to be the production phase. This is because some of the setpoints that are suitable for production of FVII are not suitable for the initial growth of the cells on the macroporous carriers. The perfusion with fresh medium at this stage is done to keep the cells alive and growing.

An outline of this aspect of the process is shown in Table 6.

**Table 6**

| **Setpoint** | **Range** | **Preferred range** | **More preferred Value** |
|---|---|---|---|
| pH | 6-8 | 6.6-7.6 | 7.0 |
| Temperature | 28-40°C | 34-38°C | 36-37°C |
| Dissolved Oxygen Ten- | 10-90% | 20-80% | 50% |
| Medium Flow Rate | | | |
| - % tank volume per day at | 10-35% volume at 0.4-1.0x10⁶ cells/ml | 25% of tank volume perfused at 0.4-1.0x10⁶cells/ml | 25% of tank volume perfused at 0.5x10⁶ cells/ml |
| - % tank volume per day at | 30-70% volume at 0.7-3.0x10⁶ cells/ml | 50% of tank volume perfused at 0.7-3.0x10⁶ cells/ml | 50% of tank volume perfused at 1.0x10⁶ cells/ml |
| - % tank volume per day | 60-95% volume at 1.0- | 80% of tank volume | 80% of tank volume |
| At | 12.0x10⁶ cells ml-1 | perfused at 1.0-12.0x10⁶cells/ml | perfused at 2.0-10x10⁶cells/ml |

### Production Phase

In the growth phase the culture is propagated until the cells reach a density of 1-12x10⁶ cells per ml. Reaching this density, the culture enters the production phase. Setpoints may also be changed at this point and set at values suitable for production of FVII. The medium perfusion is performed continuously. For the purposes of comparison the flow rate of medium is expressed in terms of percentage tank volume of medium per defined period of time. (A more standard unit would be liters per day). Medium perfusion may be from 10-200% tank volume per 10-48 hours; preferably, the medium perfusion is 80% per 10-48 hours, more preferred 80% tank volume every 24 hours.

An outline of this aspect of the process is shown in Table 7.

**Table 7**

| **Setpoint** | **Range** | **Preferred range** | **More preferred Value** |
|---|---|---|---|
| pH | 6-8 | 6.6-7.6 | 7.0 for CHO |
| Temperature | 26-40°C | 30-37°C | 32°C for CHO |
| Dissolved Oxygen Tension | 10-90% | 20-80% | 50% |
| - % tank volume of medium perfused | 10-200% of tank volume perfused in 10-48 hours | 80% of tank volume perfused in 10-48 hours | 80% of tank volume perfused every 24 hours |

### Perfusion Devices

Cell retention within the culture vessel may be achieved using a number of cell retention devices. The following sets of apparatus may all be used for this process, including, but not limited to, external settling head, internal settling head, continuous centrifuge, internal or external spin filter, external filter or hollow fiber cartridge, ultrasonic cell separating device, and a length of pipe inside the culture vessel.

Optionally, a drop in temperature may be employed when entering and/or during the production phase. In particular when using a CHO cell line, a drop in temperature is preferred. Temperature ranges and preferred values in growth and production phase, respectively, can be seen from Tables 4 and 5. A temperature of about 32°C would be preferred for a CHO cell line during the production phase.

### Batch / Draw Fill Process:

These are probably the simplest type of fermentations to operate and there are three main options for a suspension cell process using this format: 1. Simple Batch Process; 2. Fed-Batch Process, or 3. Draw-Fill Process.

### Simple Batch Process:

In a simple batch process the cells are inoculated into a seed culture vessel containing serum-free insulin-free culture medium and propagated until the cells reach a minimum density. Subsequently, the propagated seed culture is transferred to a large-scale culture vessel containing serum-free insulin-free culture medium. The culture vessel is then operated until the nutrients in the medium are exhausted. An outline of this aspect of the process is shown in Table 8.

**Table 8**

| **Setpoint** | **Range** | **Preferred range** | **More preferred Value** |
|---|---|---|---|
| pH | 6-8 | 6.6-7.6 | 7.0 |
| Temperature | 28-40°C | 30-37°C | 36-37°C |
| Dissolved Oxygen Tension | 10-90% | 20-80% | 50% |
| - Temperature drop to (Optional) | 26-39°C | 30-36°C | 32°C |
| - Temperature drop at | 0.5 - 12.0x10⁶ cells/ml | 0.5 - 12.0x10⁶ cells/ml | 2.0-10x10⁶ cells/ml |

An optional aspect of the process is the use of a reduced operating temperature. Such a batch process would consist of an initial growth phase at a specific temperature suitable for growth of the used cell line followed by a drop in operating temperature at a predetermined cell density, for example 1-12x10⁶ cells ml. This is particularly relevant for CHO cell lines. A preferred batch process for CHO would consist of an initial growth phase at 37°C followed by a drop in operating temperature at 1-12x10⁶ cells/ml, preferably 2-10x10⁶ cells/ml. Preferably, the temperature drop would be from 37°C to 32°C in case of CHO cells. The time of harvest has to be determined. A traditional batch is operated until all nutrients become exhausted. However, this typically causes cell lysis, which either can be damaging to the product or may cause problems to purification.

### Fed-Batch Process:

As stated previously a simple batch process consists inoculating a culture vessel with cells and operating the tank until the nutrients in the medium are exhausted. A batch process such as this can be extended by feeding a concentrated solution of nutrients to the tank. This extends the process time and ultimately leads to an increase in FVII production within the culture vessel.

It is important to monitor the glucose concentration in the culture vessel. The control and initiation of the feed is linked to the level of this nutrient. When the glucose concentration falls below a critical value a feed is initiated and the amount of feed added is sufficient to raise the glucose concentration back to this critical value. An outline for a Fed-batch aspect process is shown in Table 9.

**Table 9**

| **Setpoint** | **Range** | **Preferred range** | **More preferred Value** |
|---|---|---|---|
| pH | 6-8 | 6.6-7.6 | 7.0 |
| Temperature | 28-40°C | 30-37°C | 36-37°C |
| Dissolved Oxygen Ten- | 10-90% | 20-80% | 50% |
| | | | |
| - Temperature drop to (Optional) | 26-39°C | 30-36°C | 32°C |
| - Temperature drop at | 0.5 - 12.0x10⁶ cells/ml | 0.5 - 12.0x10⁶ cells/ml | 2.0-10x10⁶ cells/ml |
| Feed initiated at glucose concentration | 6-0 g/l | 3-0 g/l | When glucose < 2 g/l |

Again it may be useful to use a reduced operating temperature particularly when growing CHO cell lines. Such a fed batch process would consist of an initial growth phase at a specific temperature suitable for growth of the used cell line followed by a drop in operating temperature at a predetermined cell density, for example 1-12x10⁶ cells/ml. A preferred fed batch process for CHO would consist of an initial growth phase at 37°C followed by a drop in operating temperature at 1-12x10⁶ cells/ml, preferably 2-10x10⁶ cells/ml. Preferably, the temperature drop would be from 37°C to 32°C in case of CHO cells.

Like in a simple batch process the time of harvest has to be determined as a balance between the longest possible operation of the tank and the risk of cell lysis.

### Feed Composition & Addition Strategy

The simplest feed suitable for use would be a concentrated solution of glucose. However, glucose feeding alone will only extend the batch phase for a short length of time. This is because another nutrient such as an amino acid or lipid or vitamin will then become exhausted. For this reason a concentrated feed would be preferable. The simplest concentrated feed suitable for use would be the cell medium at a x10-50 concentration.

An outline of this aspect is shown in Table 10.

**Table 10**

| **Feed Compositions** | **Range** | **Preferred range** | **More preferred Value** |
|---|---|---|---|
| Glucose | 50-1000 g/l | 50-500 g/l | 200 g/l |
| Medium Concentrate | X 10-50 | X 2-20 | X10 |
| Modified concentrate so individual medium components are in the ranges of: | 0 -x50 | 0 -x20 | 0 - x 10 |
| Most Probable of a Composition of a Concentrate | | | |
| • Buffer | 0 -x50 | 0-x20 | x1 |
| • Lipids | 0 -x50 | 0 -x20 | x1 |
| • Iron source | 0 -x50 | 0 -x20 | x1 |
| • Cysteine and Cysteine | 0 -x50 | 0 -x20 | x1 |
| • Plant hydrolysates | 0 -x50 | 0 -x20 | x1 |
| • All other components | 0-x50 | 0 -x20 | x10 |

Unfortunately, some of the medium components may be detrimental to the cell or simply will not dissolve at a high concentration. For this reason the feed might need modification to keep these problem components at a low level. The method of addition of the feed is also a variable. The feed can be added either as a single pulse (once, twice, three times etc., a day) or can be fed gradually throughout a 24-hour period. An advanced feed option would be to have some form of glucose sensor glucose sensor in the culture vessel that will control the feed rate to maintain a constant glucose concentration in the vessel. The time of harvest has to be determined. A traditional, or simple, batch is operated until all nutrients become exhausted. This is not generally a problem in a Fed-Batch system. However, the process cannot be sustained indefinitely due to the accumulation of toxic metabolites. This leads to a decrease in cell viability and ultimately cell lysis. This may cause damage to the product or cause problems to subsequent purification.

### Draw-Fill Process:

Two types of Draw-Fill will be described here. These are Simple Draw-Fill and Fed Batch Draw-Fill.

### Simple Draw-Fill:

This process closely resembles a repeated batch fermentation. In batch fermentation the cells grow in the culture vessel and the medium is harvested at the end of the run. In a Draw-Fill process the culture vessel is harvested before any of the nutrients become exhausted. Instead of removing all of the contents from the vessel, only a proportion of the tank volume is removed (typically 80% of the tank volume). After the harvest, the same volume of fresh medium is added back to the vessel. The cells are then allowed to grow in the vessel once more and another 80% harvest is taken a set number of days later. In epeated batch processes the cells left in the vessel after a harvest may be used as the inoculum for the next batch.

An outline for a Draw-Fill process is shown in Table 11. The process is operated in two phases. The first phase of the process is operated identically to a simple batch process. After the first harvest, the culture vessel is again operated as a simple batch process; however, the length of the batch is shorter than the first batch because of the higher initial cell density. Theses short 'repeated batch phases' are continued indefinitely.

A simple outline of a draw-fill to be employed is:

### Initial Batch Phase

i. Inoculate vessel and allow cells to grow at a temperature suitable for growth.
ii. Drop temperature to a temperature suitable for expression at a predetermined cell density.
iii. 7 days after inoculation remove a predetermined, e.g. 80%, of the tank volume and replace with the same volume of fresh medium.

### Repeated Batch Phase

iv. Increase temperature to a temperature suitable for growth and allow the cells to grow.
v. Drop temperature to a temperature suitable for expression at a predetermined cell density.
vi. 5 days after the start of this phase remove a predetermined, e.g. 80%, of the tank volume and replace with the same volume of fresh medium.
vii. Go to step iv.

In a preferred embodiment, the cell line is a CHO cell line. A simple outline of a draw-fill we might employ for a CHO cell line is:

### Initial Batch Phase

viii. Inoculate vessel and allow cells to grow at 37°C.
vix. Drop temperature to 32°C at 2-10x10⁶ cells/ml.
vx. 7 days after inoculation remove 80% of the tank volume and replace with the same volume of fresh serum-free insulin-free medium.

### Repeated Batch Phase

xi. Increase temperature to 37°C and allow the cells to grow.
xii. Drop temperature to 32°C at 2-10x10⁶ cells/ml.
xii. 5 days after the start of this phase remove 80% of the tank volume and replace with the same volume of fresh serum-free insulin-free medium.
xiii. Go to step xi.

The culture vessel may be operated within a broad range of cycle times and a broad range of draw-fill volumes. Ranges and preferred values can be seen from Table 11.

**Table 11**

| **Setpoint** | **Range** | **Preferred Range** | **More preferred range** |
|---|---|---|---|
| Initial Batch Phase | | | |
| pH | 6-8 | 6.6-7.6 | 7.0 for CHO |
| Temperature | 28-40°C | 30-37°C | 37°C for CHO |
| Temperature drop (OPTIONAL) | | | |
| • Temperature drop to | 26-39°C | 30-36°C | 32°C |
| • Temperature drop at | 0.5 - 12.0x10⁶ cells/ml | 0.5 - 12.0x10⁶ cells/ml | 2.0-10x10⁶ cells/ml |
| DOT | 10-100% | 20-60% | 30% |
| Harvest | | | |
| • Tank volume | 10-99% | 10-90% | 80% |
| • Harvest volume | 2-10 days. | 5-10 days. | 9 days after start |
| Feed initiated | 6-0/g/l | 3-0/g/l | When glucose < 2/g/l |

| **Repeated Batch Phases** | | | |
|---|---|---|---|
| pH | 6-8 | 6.6-7.6 | 7.0 for CHO |
| Temperature | 28-40°C | 30-37°C | 37°C for CHO |
| Temperature drop (OPTIONAL) | | | |
| - Temperature drop to | 26-39°C | 30-36°C | 32°C |
| - Temperature drop at | 0.5 - 12.0x10⁶ cells/ml | 0.5-12.0x10⁶ cells/ml | 2.0-10x10⁶ cells/ml |
| DOT | 10-100% | 20-60% | 30% |
| Harvest | | | |
| • Tank volume | 10-99% | 10-90% | 80% |
| • Harvest volume | 1-7 days. | 1-7 days. | 5 days after harvest |
| Feed initiated | 3-0/g/l | 3-0/g/l | When glucose < 2/g/l |

### Fed-Batch Draw Fill

This process is a draw-Fill fermentation with a concentrated feed similar to the type proposed in the fed-batch process. A concern with a simple draw-fill process is that the fresh medium added may not be sufficient to sustain the cells over repeated batch fermentations. The inclusion of a feed would remove this worry. A feed would also allow operating the culture vessel with long batch times in a draw-fill process.

The composition of the feed and the strategy for addition would be identical to that of the fed-batch process. A process outline for this is shown in Table 12.

**Table 12**

| **Setpoint** | **Range** | **Preferred range** | **More preferred Value** |
|---|---|---|---|
| **Initial Batch Phase** | | | |
| pH | 6-8 | 6.6-7.6 | 7.0 for CHO |
| Temperature | 28-40°C | 30-37°C | 37°C for CHO |
| Temperature drop (OPTIONAL) | | | |
| - Temperature drop to | 26-39°C | 30-36°C | 32°C |
| - Temperature drop at | 0.5 - 12.0x10^{b} cells/ml | 0.5 - 12.0x10⁶ cells/ml | 2.0-10x10⁶ cells/ml |
| DOT | 10-100% | 20-60% | 30% |
| Harvest | | | |
| • Tank volume | 10-99% | 10-90% | 80% |
| • Harvest time | 2-10 days | 5-10 days. | 9 days after start |
| Feed initiated | 6-0 g/l | 3-0 g/l | When glucose < 2 g/l |

| **Repeated Batch Phases** | | | |
|---|---|---|---|
| pH | 6-8 | 6.6-7.6 | 7.0 for CHO |
| Temperature | 28-40°C | 30-37°C | 37°C for CHO |
| Temperature drop (OPTIONAL) | | | |
| - Temperature drop to | 26-39°C | 30-36°C | 32°C |
| - Temperature drop at | 0.5 - 12.0x10⁶ cells/ml | 0.5 - 12.0x10⁶ cells/ml | 2.0-10x10⁶ cells/ml |
| DOT | 10-100% | 20-60% | 30% |
| Harvest | | | |
| • Tank volume | 10-99% | 10-90% | 80% |
| • Harvest time | 1-7 days. | 1-7 days. | 5 days after harvest |
| Feed initiated | 5-0 g/l | 3-0 g/l | When glucose < 2 g/l |

### EXAMPLES

### Example 1: Isolation of CHO K1 cell line expressing Factor VII in serum-free insulin-free conditions

A Chinese Hamster Ovarian (CHO) cell line 1E9 that was addicted to human recombinant insulin (rInsulin) for cell growth was serially cultured as follows to develop a cell line able to produce FVII in insulin-free serum-free conditions. The cell line addicted to human recombinant insulin growth was CHO rFVIIa BI WCB07002 deposited as Accession number 08100801 with the European Collection of Cell Cultures, Porton Down, U.K., on October 8, 2008. Media used was BCS medium supplemented with Vitamin K1 (2.5 mg/ml) [Merck] and serial diluted amounts of rInsulin [Insulin recombinant, NOVOLIN, Novo Nordisk].

"Insulin-free" media was serum-free BCS media containing 2.5 mg/l Vitamin K1. For the serial dilutions, BSC media containing serially dilutions of rInsulin were made as described in Table 13, including 1.25 mg/l, 0.625 mg/l, 0.208 mg/l, 0.069 mg/l, 0.250 mg/l, 0.063 mg/l, 0.031 mg/l and 0.015 mg/l.

**Table 13**

| Name Media | Description | Composition |
|---|---|---|
| M/CBL/07/081 | Insulin-free | BCS Media (ORSFBCS0700102) Vitamin K1 (2.5 mg/ml) |
| M/CBL/07/083 | 1.25 mg/l rInsulin | BCS Media (ORSFBCS0700102) Vitamin K1 (2.5 mg/l) R/CBL/07/023 rInsulin Human (1.25 mg/l): R/CBL/07/021 |
| M/CBL/07/086 | 0.625 mg/l rInsulin | ½ M/CBL/07/081 ½ M/CBL/07/083 |
| M/CBL/07/090 | 0.208 mg/l rInsulin | ½ M/CBL/07/081 |
| | | ½ M/CBL/07/091 |
| M/CBL/07/091 | 0.625 mg/l rInsulin | ½ M//07/081 |
| | | ½ M/CBL/07/083 |
| M/CBL/07/095 | 0.069 mg/l rInsulin | ½ M/CBL/07/081 |
| | | ½ M/CBL/07/090 |
| M/CBL/07/098 | 0.25 mg/l rInsulin | M/CBL/07/081 rInsulin Human (0.250 mg/l) |
| M/CBL/07/099 | 0.063 mg/l rInsulin | ½ M/CBL/07/081 |
| | | ½ M/CBL/07/098 |
| M/CBL/07/100 | 0.031 mg/l rInsulin | ½ M/CBL/07/081 |
| | | ½ M/CBL/07/099 |
| M/CBL/07/104 | Insulin-free | BCS media (ORSFBCS0700103) |
| | | Vitamin K1 (2.5 mg/l) (R/CBL/07/023) |
| M/CBL/07/110 | Insulin-free | BCS media (ORSFBCS0700201) |
| | | Vitamin K1 (2.5 mg/l) (R/CBL/07/023) |
| M/CBL/07/118 | Insulin-free | BCS media (ORSFBCS0700301) |
| | | Vitamin K1 (2.5 mg/l) (R/CBL/07/023) |

The protocol for successful adaption of 1E9-CHO rFVIIa cells (rInsulin Human 5 mg/l-DMCB #10 (HHü07082) to serum-free insulin-free conditions was preformed by serial passaging the cells as described in Table 14. Serial passaging was performed by controlled "gradient reduction" of insulin concentration in the media until serum-free conditions were achieved. Cells were "pulsed" - with gradually reduced insulin-containing media in between culturing of cells in insulin-free media for one day. Concurrent monitoring of cell growth and viability was performed. Fig. 1 depicts the reduction of cells dependence on insulin.

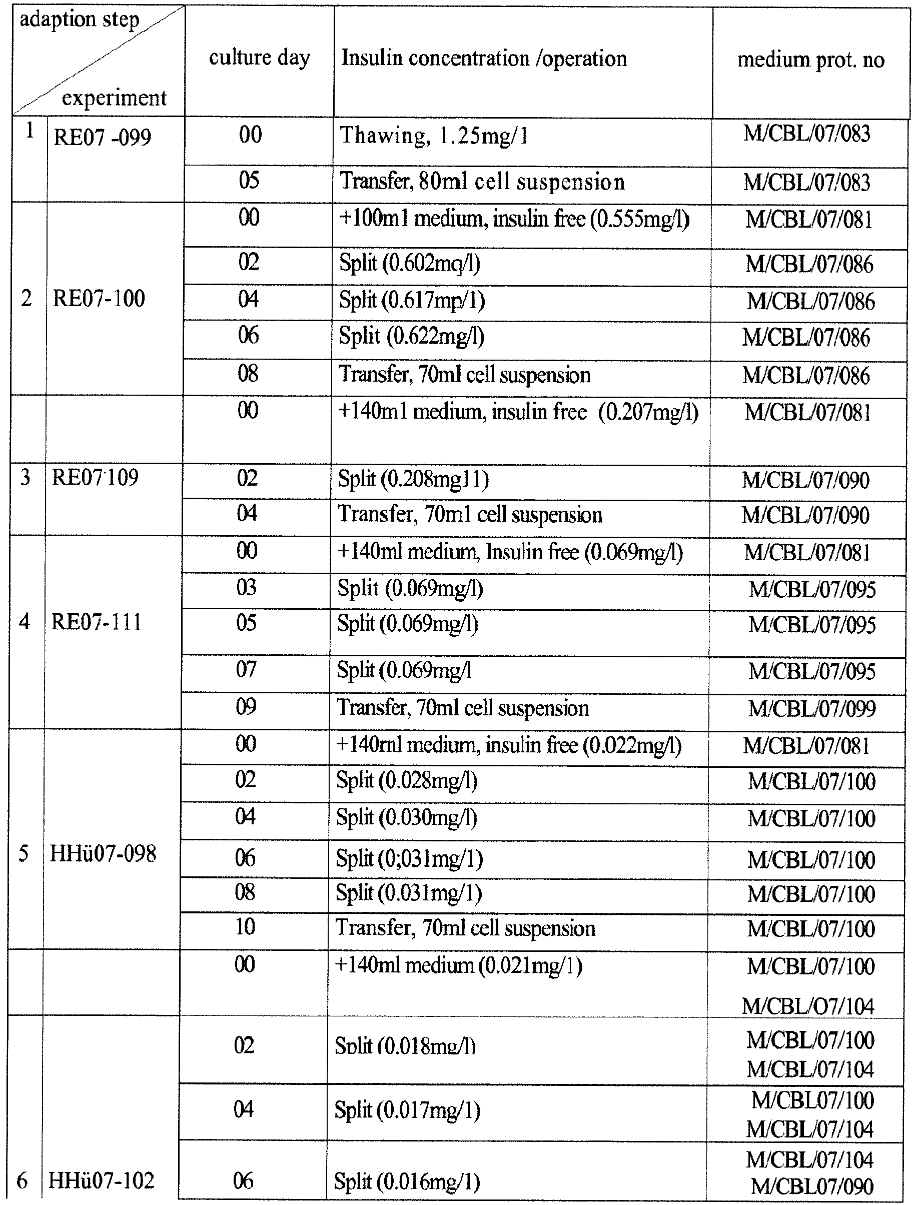

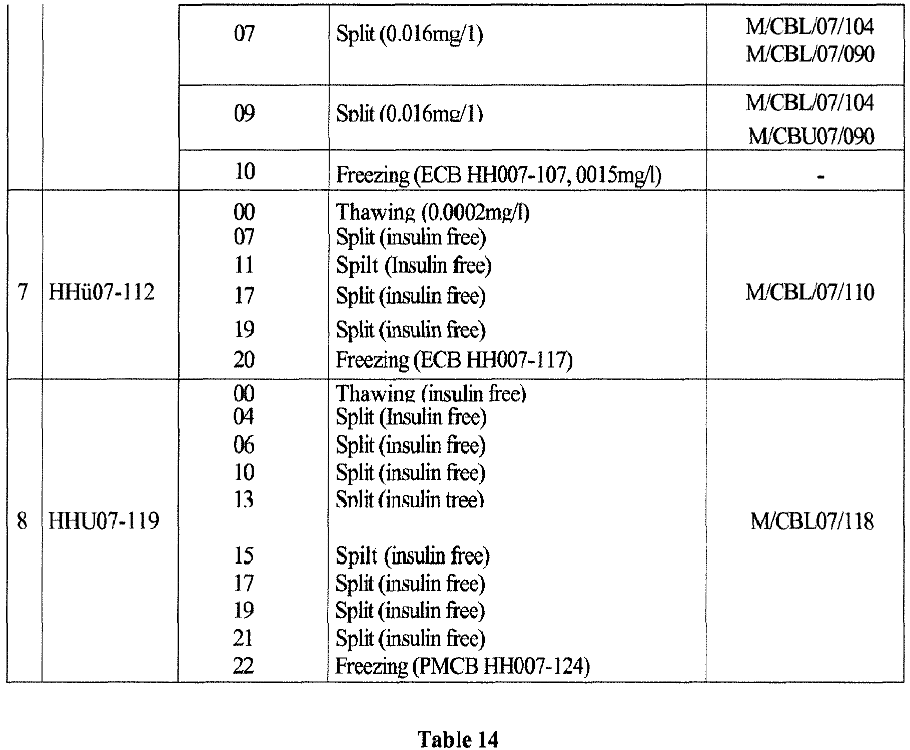

Fig. 2 depicts a graph showing the insulin-levels during pulsing of the cells to adapt to serum free conditions.

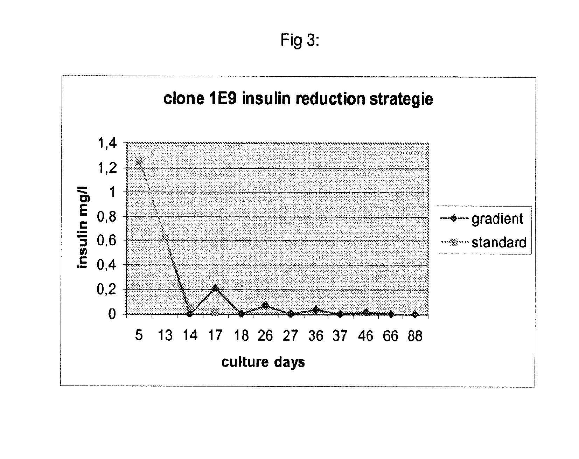

Surprisingly, this method of pulsing with insulin-containing serum-free media contains gradually decreasing amounts of insulin results in the production of a cell line that grows under serum-free insulin-free conditions, while standard passaging in media with serial dilutions of insulin does not produce a viable cell line. As shown in Fig. 3, cells passaged by standard serial dilution of rInsulin techniques did not survive beyond three passages (day 17, 0.156 mg/l insulin).

### Example 2 Exemplary Production Conditions

The rFVII is generated by a recombinant Chinese Hamster Ovary (CHO) cell clone in a fermentation process in suspension culture. The growth medium is a chemically defined medium that is free of human or animal derived substances and is free of insulin. The cell line produced as described above is cultured in commercially available tissue culture flasks and glass bottles (spinner cultures) at a temperature of 37°C. The pH of the culture is maintained by introduction of CO₂ (7.5%) and NaHCO₃ derived from the liquid medium.

A serum-free medium (BCS) is used for the cultivation of the cells. BCS medium is DMEM/HAM's F12 based formulation supplemented with the following additives: i) glutamine; final concentration 0.9 g/l, ii) ferric sulfate x7H2O 0.0006 g/l, iv) putrescine, x 2HCl 0.0036g/l, vi) Vitamin Kl 0.0025 g/l and vii) Synperonic; final concentration 1 g/l, Phenolred 0.008 g/l, Ethanolamine 0.00153g/l, Na-hydrogencarbonate 2g/l). For comparative purposes the cell line grown in serum-free, insulin-containing medium is grown in the same media but supplemented with Insulin 0.005 mg/l. Note that in this media, Vitamin K1 or K3 can be used.

The process parameter set points used for fermentation are summarized in the following Table 15:

**Table 15**

| Parameter | Set Point |
|---|---|
| Cell density | 1.0 - 3.0 x 10⁶/ml |
| Temperature | 36.0 +- 2.0 °C |
| pH | 6.8 - 7.4 |
| PO₂ | 20 % - 80% air saturation |
| Total process time (harvest) | 20- 60 days |

More specifically, the cell density of the cells in the large scale culture is at 1-3x10⁶/ml, and the culture is maintained at pH 7.1 +-0.3, pO2 10-50%, with a dilution rate of 0.25-1.5.

For the large-scale production of recombinant Factor VIIa a 100 liter bioreactor system is used for the development of pre-clinical material. A robust and stable process has been confirmed at the large scale production levels using a cell line (1E9; deposited under Accession No. number 08100801 deposited with the European Collection of Cell Cultures, Porton Down, U.K., on October 8, 2008) that grows in the presence of insulin. A data summary of the pre-clinical production campaign is given below: The specific growth rate of the cells is approx. 0.50 per day. The cell density set point at around 2 x 10⁶ cells per ml. The Factor VII antigen titer is around 10µg/. The FVII activity (chromogenic assay) is around 20 U/ml. The FVII productivity based on antigen is 3-4 mg/L/day and based on activity it is 7000 - 10000 U/L/day. Throughout the production lots the stability was consistent over a 40 day period. The inventors have shown that the cell lines produced in insulin free conditions (cell line deposited under Accession No. number 08100802 deposited with the European Collection of Cell Cultures, Porton Down, U.K., on October 8, 2008) show the same features as the original 1E9 (cultured with insulin) production cell bank. No difference could be observed in terms of specific growth rate, rFVII expression and stability of the cells.

A preferred method for culturing the cells in serum free, insulin free media is the chemostat method. This method is well known to those of skill in the art and has been described in e.g., Werner et al. J. Biotechnol. 1992, 22 pp 51-68. For example a stirred bioreactor or an airlift reactor may be used.

The parameters for culturing the cells such as 02 concentration, perfusion rate, or medium exchange, pH, temperature and culturing technique will depend on the individual cell types used and may be determined by the skilled person. For example, in specific examples, the conditions in a preferred method are such that cell density of the cells in the large scale culture is at 1-3x10⁶/ml, and the culture is maintained at pH 7.1 +-0.3, pO2 10-50%, with a dilution rate of 0.25-1.5.

All reference to herein are hereby incorporated by reference in their entirety.

The present technology is now described in such full, clear and concise terms as to enable a person skilled in the art to which it pertains, to practice the same. It is to be understood that the foregoing describes preferred embodiments of the present technology and that modifications may be made therein without departing from the spirit or scope of the present technology as set forth in the appended items. Further the examples are provided to not be exhaustive but illustrative of several embodiments that fall within the scope of the items.

THE PRESENT INVENTION IS ALSO EXEMPLIFIED BY THE FOLLOWING PREFERRED EMBODIMENTS:
1. A method for producing recombinant human Factor VII (FVII) comprising:
   a) obtaining a Chinese Hamster Ovary (CHO) cell line that expresses recombinant human FVII; and
   b) culturing said CHO cell in serum-free media that lacks insulin
2. The method of item 1, wherein the production of said FVII in said CHO cell is comparable to the production of FVII in the presence of insulin.
3. The method of item 1, wherein said CHO cell is prepared by adapting a CHO cell line that expresses recombinant human FVII modified for growth in serum-free culture to grow in cell culture in the absence of insulin by serially culturing said CHO cell line in decreasing amounts of insulin to obtain a FVII-producing CHO cell line that grows in the absence of insulin.
4. The method of item 3, wherein said CHO cell line that expresses recombinant human FVII modified for growth in serum-free culture is cell line 1E9 cell line deposited under Accession No. number 08100801 with the European Collection of Cell Cultures, Porton Down, U.K., on October 8,2008.
5. The method of item 3, wherein said FVII-producing CHO cell line that grows in the absence of insulin is a 1E9 cell line deposited under Accession No. number 08100802 with the European Collection of Cell Cultures, Porton Down, U.K., on October 8, 2008.
6. The method of item 1, wherein said cell culture medium comprises vitamin K1.
7. The method of item 3, wherein said serially culturing comprises pulsing successive passages of CHO cells with decreasing concentrations of insulin.
8. The method of item 7, comprising growing a CHO cell line up to day 14 in standard serum-free media that contains insulin and at day 14 transferring said cell line to a serum-free, insulin free media.
9. The method of item 8, comprising growing said cells in a serum-free, insulin free media for 1 to 3 days and then transferring said cells into an insulin-containing media that comprises 0.2 mg/ml insulin and growing said CHO cell line in said insulin-containing media for 1 to 3 days.
10. The method of item 9, comprising, after growth for 1 to 3 days in insulin-containing media, transferring said CHO cell line into serum-free, insulin-free media.
11. The method of item 10, comprising growing said cells in a serum-free, insulin free media for 1 to 3 days and then transferring said cells into an insulin-containing media that comprises 0.070 mg/ml insulin and growing said CHO cell line in said insulin-containing media for 3 to 8 days.
12. The method of item 11, comprising, after growth for 3 to 8 days in insulin-containing media, transferring said CHO cell line into serum-free, insulin-free media.
13. The method of item 12, comprising growing said cells in a serum-free, insulin free media for 1 to 2 days and then transferring said cells into an insulin-containing media that comprises 0.030 mg/ml insulin and growing said CHO cell line in said insulin-containing media for 3 to 9 days.
14. The method of item 13, comprising, after growth for 3 to 9 days in insulin-containing media, transferring said CHO cell line into serum-free, insulin-free media.
15. The method of item 14, comprising growing said cells in a serum-free, insulin free media for 1 to 2 days and then transferring said cells into an insulin-containing media that comprises 0.016 mg/ml insulin and growing said CHO cell line in said insulin-containing media for 3 to 9 days.
16. A method of adapting a FVII-producing cell line that grows in serum-free media to a cell line that grows in serum-free, insulin free media, comprising pulsing successive passages of CHO cells with decreasing concentrations of insulin, wherein said pulsing comprises a) growing said cell line for 1 to 9 days in insulin-containing media followed by b) growth in insulin-free, serum-free media for 1 to 3 days and repeating steps a) and b) wherein the media in each successive step a) contains approximately half the concentration of insulin as the previous step a) until the media contains no detectable insulin.
17. The method of item 16, wherein said method comprises:
   a) growing a CHO cell line up to day 14 in standard serum-free media that contains insulin and at day 14 transferring said cell line to a serum-free, insulin free media;
   b) growing said cells of step b) in a serum-free, insulin free media for 1 to 2 days and then transferring said cells into an insulin-containing media that comprises 0.2 mg/ml insulin and growing said CHO cell line in said insulin-containing media for 1 to 3 days;
   c) after growth for 1 to 3 days in insulin-containing media of step b), transferring said CHO cell line into serum-free, insulin-free media;
   d) growing said cells of step c) in a serum-free, insulin free media for 1 to 3 days and then transferring said cells into an insulin-containing media that comprises 0.070 mg/ml insulin and growing said CHO cell line in said insulin-containing media for 3 to 8 days;
   e) after growth for 3 to 8 days in insulin-containing media in step d), transferring said CHO cell line into serum-free, insulin-free media;
   f) growing said cells of step e) in a serum-free, insulin free media for 1 to 2 days and then transferring said cells into an insulin-containing media that comprises 0.030 mg/ml insulin and growing said CHO cell line in said insulin-containing media for 3 to 9 days;
   g) after growth for 3 to 9 days in insulin-containing media in step f), transferring said CHO cell line into serum-free, insulin-free media;
   h) growing said cells of step g) in a serum-free, insulin free media for 1 to 2 days and then transferring said cells into an insulin-containing media that comprises 0.016 mg/ml insulin and growing said CHO cell line in said insulin-containing media for 3 to 9 days; and
   i) after growth for 3 to 9 days in insulin-containing media in step h, transferring said CHO cell line into serum-free, insulin-free media, wherein the cells in step i) are adapted for long-term growth in serum-free, insulin-free media.
18. A cell produced according to the method of item 16 or item 17.
19. A recombinant CHO cell that expresses human FVII and is adapted for growth in an insulin-free media lacking animal-derived components.
20. The recombinant cell of item 19, wherein said cell is produced by adapting a FVII-producing cell line that grows in serum-free media to a cell line that grows in serum-free, insulin free media, comprising pulsing successive passages of CHO cells with decreasing concentrations of insulin, wherein said pulsing comprises a) growing said cell line for 1 to 9 days in insulin-containing media followed by b) growth in insulin-free, serum-free media for 1 to 3 day and repeating steps a) and b) wherein the media in each successive step a) contains approximately half the concentration of insulin as the previous step a) until the media contains no detectable insulin.
21. A recombinant CHO cell that expresses human FVII deposited under Accession No. number 08100801 with the European Collection of Cell Cultures, Porton Down, U.K., on October 8, 2008.
22. A recombinant CHO cell that expresses human FVII deposited under Accession No. number 08100802 with the European Collection of Cell Cultures, Porton Down, U.K., on October 8,2008.
23. A method for large-scale production of a FVII or a FVII-related polypeptide in mammalian cells, said method comprising:
   (a) inoculating a cell according to item 18 into a culture vessel containing serum-free, insulin-free medium and propagating said mammalian cell culture at least until the cells reach a predetermined density;
   (b) transferring said propagated seed culture to a large-scale culture vessel containing serum-free insulin-free medium;
   (c) propagating said large-scale culture in serum-free insulin-free medium, at least until said cells reach a predetermined density;
   (d) maintaining the culture obtained in step (c) in serum-free insulin-free medium, under conditions appropriate for FVII expression or FVII-related polypeptide expression; and
   (e) recovering the FVII or the FVII-related polypeptide from the maintained culture.
24. The method of item 23, further comprising, prior to step (b), repeating step (a) using seed culture vessels of progressively increasing size.
25. The method of item 23, further comprising: maintaining the culture obtained in step (c) in serum-free, insulin-free media by regular harvesting of the culture medium and replacement by fresh medium.
26. The method of item 23, wherein the method is microcarrier process.
27. The method of item 26, wherein the method is a macroporous carrier process.
28. The method of item 26, wherein the method is a standard microcarrier process.
29. The method of item 26, wherein the method is a microcarrier perfusion process.
30. The method of item 23, wherein the method is a suspension process.
31. The method of item 30, wherein the method is a perfusion process.
32. A method as defined in item 30, wherein the method is a batch/draw-fill process.
33. The method of item 32, wherein the method is a simple batch process.
34. The method of item 32, wherein the method is a fed-batch process.
35. The method of item 32, wherein the method is a draw-fill process.
36. The method of item 23, wherein said method is a chemostat culturing process.
37. The method of item 23, wherein said cells, prior to said inoculating step, have been adapted to grow in serum-free medium lacking insulin.
38. The method of item 23, wherein said cells, prior to said inoculating step, are capable of growing in suspension culture.
39. The method of item 23, wherein said desired FVII or FVII-related polypeptide is human FVII or a human FVII-related polypeptide.
40. The method of item 23, wherein FVII or a FVII -related polypeptide is produced at a level at least about 1 mg/l/day of culture.
41. The method of item 40, wherein FVII or a FVII-related polypeptide is produced at a level at least about 2.5 mg/l/day of culture.
42. The method of item 41, wherein FVII or a FVII-related polypeptide is produced at a level at least about 5 mg/l/day of culture.
43. The method of item 42, wherein FVII or a FVII -related polypeptide is produced at a level at least about 8 mg/l/day of culture.
44. The method of item 42, wherein FVII or a FVII-related polypeptide is produced at a level of about 3-4 mg/l/day.
45. The method of item 42, wherein the FVII or FVII-related polypeptide units produced are at least 5000 U/l/day.
46. The method of item 42, wherein the FVII or FVII-related polypeptide units produced are at least 7000 U/l/day.
47. The method of item 42, wherein the FVII or a FVII-related polypeptide units of activity produced is about 7000-10,000 U/l/day.
48. The method of item 23, further comprising: maintaining the culture obtained in step (c) in animal product free, insulin-free media by regular harvesting of part of the culture supernatant after sedimentation of the cell-containing carriers and replacement by fresh medium
49. The method of item 48, further comprising: cooling of the culture to a pre-determined temperature below the setpoint of the cultivation before the sedimentation of carriers.
50. The method of item 49, where the culture is cooled to a temperature of from 5°C to 30°C below the temperature setpoint of the cultivation before the sedimentation of carriers.
51. The method of item 50, where the culture is cooled to a temperature of from 5°C to 20°C below the temperature setpoint of the cultivation.
52. The method of item 51, where the culture is cooled to a temperature of from 5°C to 15°C below the temperature setpoint of the cultivation.
53. The method of item 52, where the culture is cooled to a temperature of about 10°C below the temperature setpoint of the cultivation.
54. A method of item 1, item 16 or item 23, wherein said media is a serum-free DMEM/HAM's F12 based formulation supplemented with one or more of the following additives i) glutamine; final concentration 0.9 g/l, ii) ferric sulfate x7H2O 0.0006 g/l, iv) putrescine, x 2HCl 0.0036g/l, vi) Vitamin K1 0.0025 g/l, vii) Synperonic; final concentration 1 g/l, Phenolred 0.008 g/l, Ethanolamine 0.00153g/l, and Na-hydrogencarbonate 2g/l).

## Claims

1. A method for producing recombinant human Factor VII (FVII) comprising:
a) obtaining a mammalian cell line that expresses recombinant human FVII; and
b) culturing said mammalian cell in serum-free media that lacks insulin,
wherein said cell is prepared by adapting a cell line that expresses recombinant human FVII modified for growth in serum-free culture to grow in cell culture in the absence of insulin by serially culturing said cell line in decreasing amounts of insulin to obtain a FVII-producing cell line that grows in the absence of insulin,
wherein said serially culturing comprises pulsing successive passages of cells with decreasing concentrations of insulin.

2. The method of claim 1, wherein the production of said FVII in said cell is comparable to the production of FVII in the presence of insulin.

3. The method of claim 1, wherein said cell culture medium comprises vitamin K1.

4. The method of claim 1, comprising growing a cell line up to day 14 in standard serum-free media that contains insulin and at day 14 transferring said cell line to a serum-free, insulin free media.

5. The method of claim 4, comprising growing said cells in a serum-free, insulin free media for 1 to 3 days and then transferring said cells into an insulin-containing media that comprises 0.2 mg/ml insulin and growing said cell line in said insulin-containing media for 1 to 3 days.

6. The method of claim 5, comprising, after growth for 1 to 3 days in insulin-containing media, transferring said cell line into serum-free, insulin-free media.

7. The method of claim 6, comprising growing said cells in a serum-free, insulin free media for 1 to 3 days and then transferring said cells into an insulin-containing media that comprises 0.070 mg/ml insulin and growing said cell line in said insulin-containing media for 3 to 8 days.

8. The method of claim 7, comprising, after growth for 3 to 8 days in insulin-containing media, transferring said cell line into serum-free, insulin-free media.

9. The method of claim 8, comprising growing said cells in a serum-free, insulin free media for 1 to 2 days and then transferring said cells into an insulin-containing media that comprises 0.030 mg/ml insulin and growing said cell line in said insulin-containing media for 3 to 9 days.

10. The method of claim 9, comprising, after growth for 3 to 9 days in insulin-containing media, transferring said cell line into serum-free, insulin-free media.

11. The method of claim 10, comprising growing said cells in a serum-free, insulin free media for 1 to 2 days and then transferring said cells into an insulin-containing media that comprises 0.016 mg/ml insulin and growing said cell line in said insulin-containing media for 3 to 9 days.

12. A method of adapting a FVII-producing cell line that grows in serum-free media to a cell line that grows in serum-free, insulin free media, comprising pulsing successive passages of mammalian cells with decreasing concentrations of insulin, wherein said pulsing comprises a) growing said cell line for 1 to 9 days in insulin-containing media followed by b) growth in insulin-free, serum-free media for 1 to 3 days and repeating steps a) and b) wherein the media in each successive step a) contains approximately half the concentration of insulin as the previous step a) until the media contains no detectable insulin.

13. The method of claim 12, wherein said method comprises:
a) growing a mammaliancell line up to day 14 in standard serum-free media that contains insulin and at day 14 transferring said cell line to a serum-free, insulin free media;
b) growing said cells of step b) in a serum-free, insulin free media for 1 to 2 days and then transferring said cells into an insulin-containing media that comprises 0.2 mg/ml insulin and growing said cell line in said insulin-containing media for 1 to 3 days;
c) after growth for 1 to 3 days in insulin-containing media of step b), transferring said cell line into serum-free, insulin-free media;
d) growing said cells of step c) in a serum-free, insulin free media for 1 to 3 days and then transferring said cells into an insulin-containing media that comprises 0.070 mg/ml insulin and growing said cell line in said insulin-containing media for 3 to 8 days;
e) after growth for 3 to 8 days in insulin-containing media in step d), transferring said cell line into serum-free, insulin-free media;
f) growing said cells of step e) in a serum-free, insulin free media for 1 to 2 days and then transferring said cells into an insulin-containing media that comprises 0.030 mg/ml insulin and growing said cell line in said insulin-containing media for 3 to 9 days;
g) after growth for 3 to 9 days in insulin-containing media in step f), transferring said cell line into serum-free, insulin-free media;
h) growing said cells of step g) in a serum-free, insulin free media for 1 to 2 days and then transferring said cells into an insulin-containing media that comprises 0.016 mg/ml insulin and growing said cell line in said insulin-containing media for 3 to 9 days; and
i) after growth for 3 to 9 days in insulin-containing media in step h, transferring said cell line into serum-free, insulin-free media, wherein the cells in step i) are adapted for long-term growth in serum-free, insulin-free media.

14. A cell obtainable by the method of claim 12 or claim 13.

15. A recombinant mammalian cell that expresses human FVII and is adapted for growth in an insulin-free media lacking animal-derived components, wherein said cell is obtainable by adapting a FVII-producing cell line that grows in serum-free media to a cell line that grows in serum-free, insulin free media, comprising pulsing successive passages of mammalian cells with decreasing concentrations of insulin, wherein said pulsing comprises a) growing said cell line for 1 to 9 days in insulin-containing media followed by b) growth in insulin-free, serum-free media for 1 to 3 day and repeating steps a) and b) wherein the media in each successive step a) contains approximately half the concentration of insulin as the previous step a) until the media contains no detectable insulin.

16. A method for large-scale production of a FVII or a FVII-related polypeptide in mammalian cells, said method comprising:
(a) inoculating a cell according to claim 14 into a culture vessel containing serum-free, insulin-free medium and propagating said mammalian cell culture at least until the cells reach a predetermined density;
(b) transferring said propagated seed culture to a large-scale culture vessel containing serum-free insulin-free medium;
(c) propagating said large-scale culture in serum-free insulin-free medium, at least until said cells reach a predetermined density;
(d) maintaining the culture obtained in step (c) in serum-free insulin-free medium, under conditions appropriate for FVII expression or FVII-related polypeptide expression; and
(e) recovering the FVII or the FVII-related polypeptide from the maintained culture.

17. The method of claim 16, wherein said desired FVII or FVII-related polypeptide is human FVII or a human FVII-related polypeptide.

18. A method of claim 1, claim 12 or claim 16, wherein said media is a serum-free DMEM/HAM's F12 based formulation supplemented with one or more of the following additives i) glutamine; final concentration 0.9 g/l, ii) ferric sulfate x7H2O 0.0006 g/l, iv) putrescine, x 2HCl 0.0036g/l, vi) Vitamin K1 0.0025 g/l, vii) Synperonic; final concentration 1 g/l, Phenolred 0.008 g/l, Ethanolamine 0.00153g/l, and Na-hydrogencarbonate 2g/l).

19. The method, cell or recombinant cell of any one of the preceding claims, wherein the cells are BHK cells, HEK293 cells or COS-1 cells.
